# EUROPEAN PATENT APPLICATION

(11) **EP 4 459 629 A1**
(43) Date of publication of application: **06.11.2024**
(21) Application number: 23177853.1
(22) Date of filing: 07.06.2023
(51) Int. Cl.: G16H 20/30, G16H 50/70

(54) **CLOUD ANALYSIS SYSTEM FOR SPORT AND HEALTH**

(30) Priority: 04.05.2023 TW 112116647
(71) Applicant: Decentralized Biotechnology Intelligence Co., Ltd., Taipei City, Da'an Dist. 106021 (TW)
(72) Inventor: Chou, Yao-Sheng, 106021 Taipei (TW); Wu, Chung-Yuan, 106021 Taipei (TW); Chou, Yen-Han, 106021 Taipei (TW)
(74) Representative: Zaboliene, Reda

(57) **Abstract**

The present invention discloses a sports and health cloud analysis system including a cloud server with an intelligent analysis module, a big data database is coupled to the cloud server, a wearable sensing module is coupled to a mobile device for collecting at least heart sound, ECG, lung sounds, blood pressure, blood glucose level and blood oxygen saturation level information; an insole-type sensing module is used to collect at least foot information, which is connected to the mobile device. The sensing module performs at least one of exercise sensing, foot, exercise, ankle exercise, knee exercise, hand exercise. The collected information is analyzed by an intelligent analysis module to obtain data for evaluating the health status and exercise intensity.

## Description

### TECHNICAL FIELD

The present invention relates to an analysis system, more specifically, a cloud analysis system for sport and health.

### BACKGROUND

Heart failure is a serious health issue in the world. In recent years, the physical and mental status monitoring has become popular with the paces of technology and the rise of people's health awareness. The cardiac muscle contraction vibrations may be detected on the chest through the surrounding tissues. When sensors are placed near the chest, the detected sound is generally called heart sound. The cardiac activity is typically diagnosed by heart sounds. The electronic stethoscope is a device for collecting heart and lung organ activities, allowing doctors to determine the cause of the disease based on the sound signal. By monitoring physiological information, it effectively improves early diseases detection and health management. The physiological information includes heart rate, ECG waveform and others. In order to detect symptoms, especially diseases with sudden death, the doctors have to analyze the physiological information and provide health care solutions based on the detected data.

In addition, plantar pressure distributions are important indexes to reveal human gait, the gait analysis is a theory to analyze human daily walking status, and the plantar pressure distribution provides significant information for the fields of biomechanics, rehabilitation, physical training, and shoemaking. The pressure testing boards and benches currently used in clinical practices have space limitations and are not likely wearable. Conventional plantar pressure sensors are in contact with the foot, frequently. Therefore, it is prone to wear and tear due to the sensors contact with the sole of the foot. It is not conducive for long-term wearing and testing, and it cannot provide sufficient health information.

With the rapid development of cloud computing, wireless communication and artificial intelligence, a health monitoring system integrating various sensors and intelligent computing has become the trends of research. The wearable heart sound device may provide real-time heartbeat detection and recording, it is desired to provide an advanced system for evaluating health status and exercise intensity. Therefore, in view of this, what is required is to provide a modern monitoring system to collect health and sport information, simultaneously.

### SUMMARY OF THE INVENTION

Based on above, one aspect and purpose of the present invention is to provide a sport and health cloud system. The sport/health cloud system of the present invention completely evaluates the health status and exercise intensity, and also moderates exercise and training according to the individual's physical condition to reduce the risk of injury and take care of health, simultaneously. The processed health status and exercise intensity may also be share and used by third parties to achieve the synergic effect of cross-industries combination.

In one aspect, the sport/health cloud system includes an intelligent analysis module; a big data database is electrically coupled to the cloud server. A wearable sensing device is employed for collecting at least one of heart sounds, electrocardiograms, lung sounds, blood pressure, blood glucose level and blood oxygen saturation level. An insole-type sensing device is introduced for collecting at least foot information; and wherein the wearable sensing device and the insole sensing device are connected to a mobile device connected to the cloud server for transmitting collected data and subsequently analyzed by the intelligent analysis module to obtain health or exercise data for the assessment of health status or exercise intensity.

The wearable sensing device includes an internet of things (IoT) wearable stethoscope which is connected to the mobile device for real-time monitoring on line, or recording heart sound information offline. The mobile device displays analyzed heart sound information which is indicated with a normal, an abnormal or an emergency state, and the mobile device issues a warning signal for the abnormal and the emergency states. The mobile device is set in advance with an emergency contact person, an ambulance service, a police department or an insurance company for designating medical care unit, emergency contact, emergency rescue or rescue network.

The insole-type sensing device collects a foot information which is displayed in real time by the mobile device to analyze a personal movement or exercise, and establish a relationship between the exercise and a foot pressure. The insole-type sensing device includes a pressure sensor, an infrared sensor, an accelerometer, a gyroscope, a GPS or any combination thereof. The analyzed data includes one or any combination of following: a foot pressure distribution, a weight ratio of the feet, a gait, a stride frequency, and a pressure center.

The data stored in the mobile device or the big data database is used for health management or used by a third party. The third party includes, but not limit to, a hospital, an insurance companies, a senior person center or a brand manufacturer. The big data database employs a block chain technology. Therefore, the data cannot be changed and the data is encrypted during transmission.

In another aspect of the present invention, the present invention can achieve precise exercise measurement, whether it is uphill or downhill exercise, through accurate sensing data, it is beneficial to classify exercise intensity. The present invention provides exercise sensing and management, it also provides details of each course of exercise, and can be compared with past records in different places to improve the training effect. The present invention can detect heart sounds for 24 hours, the sensor can be worn for 24 hours/365 days to record the state of heart sounds, AIoT (AI and IoT) technology is used to track health data analysis, monitor heart rate changes, and issue emergency warning.

Based on above, the present invention proposes to solve the existing deficiencies of the prior art. According to one aspect of the present invention, the wearable sensing device is proposed to collect at least one of following signal: heart sounds, electrocardiograms, and lung sounds of the body, blood pressure, blood glucose level and blood oxygen saturation level and other. The sensing devices of the present invention communicate with the big data database via an external computing electronic device.

In one embodiment, the AI comparison and status classification is performed using normal signals and abnormal signals by AI algorithm installed in an external computing electronic device. Preferably, the AI algorithm may perform the following steps: pre-filtering and normalizing the input heart sound signal; extracting time-domain and frequency-domain features from the processed filtering and normalizing heart sound signal; Convolutional Neural Networks (CNN) model is used to output classification results.

### BRIEF DESCRIPTION OF THE DRAWINGS

Figure 1 shows the cloud system of the present invention.
Figure 2 shows the sensing devices functional diagram of the present invention.

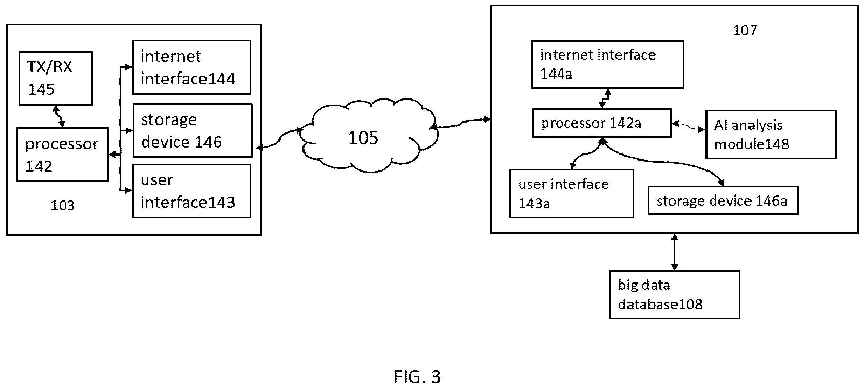

Figure 3 shows the cloud system and the sensing device functional diaphragms of the present invention.
Figure 4 shows the cloud system with the third parties according to one embodiment of the present invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The present invention integrates the internet of things (IoT), artificial intelligence (AI) and dynamic sensing technology. The present invention allows users to wear the sensors without uncomfortable feeling, but it is able to accurately record and analyze exercise status, and provide users with the most complete exercise information as well. The present invention offers real-time feedback of sport information by APP, it is not only helpful for sport management, but also can analyze various sports characteristics by sport history, completely evaluate the sport status and fitness to reduce the risk of injury and improve sport efficiency. Sport management made easier and more fun, the present invention provides an indispensable tool for performing sport management.

The present invention discloses a wearable heart sound detection system including a wearable heart sound sensing device worn on the human body. The sound sensing device integrates with a wireless transmission device to connect with the loT. The collected physiological data (for example, personal heart sound) is processed through a handheld electronic computing device (such as mobile device), and the processed data is sent and stored in a cloud server through a cloud network. The plantar pressure detection system of the present invention is composed of a plurality of pressure sensing elements. During the measurement process, the pressure value is collected and calculated according to each sensing element to obtain the measured plantar pressure parameter.

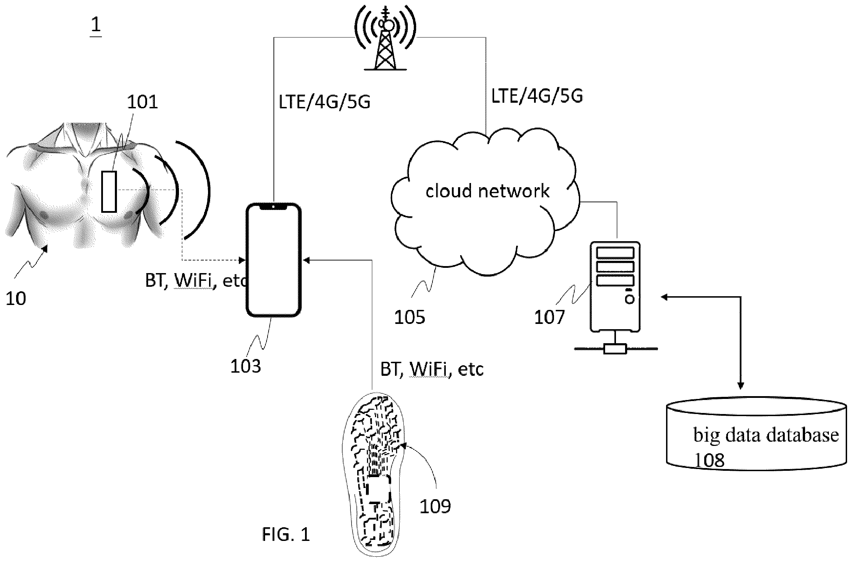

FIG. 1 shows a sport and health cloud analysis system 1 of the present invention, the system 1 includes a cloud server 107 electrically connected to a big data database 108. The wearable sensing device 101 detects physiological data, and detects various types of exercise measurement as well, such as at least one of the following: foot exercise, ankle exercise, knee exercise, and hand exercise sensing measurement. Various exercise sensing device includes at least a foot sensing device (such as an insole sensor) or a wearable sensing device (such as a kneepad sensor). The exercise information is collected by using the wearable sensing device or the insole sensing device, wherein the exercise includes at least one of foot and hand exercise. The wearable sensing device 101 may be configured on any suitable body position, such as the neck, head, shoulder, arm, wrist, thigh, calf, ankle, cardiopulmonary area and so on. According to the configuration and arrangement of the wearable sensing device 101, the physiological health detection may include at least one of heart sound, electrocardiogram, lung sound, blood pressure, blood glucose level and blood oxygen saturation level and the like.

According to a preferred embodiment, the wearable sensing device 101 can be used to detect not only heart sounds, but also at least one of Electrocardiography (ECG), lung sounds, blood pressure, blood glucose level and blood oxygen saturation level and the like. For example, according to the sensor configuration of the wearable sensing device 101, ECG can be detected. For example, the heart activity reveals human body information including emotional state and early diagnosis of heart disease. By changing the type of the wearable sensing device 101, such as an ECG sensor or a Photoplethysmography (PPG) sensor, different signals may be detected. Electrodes are connected to measure the electrical activity signal induced by the heart tissue. With the heartbeat, there is pressure waves passing through the blood vessels. This wave changes the diameter of the blood vessels. ECG is used to measure related parameters. PPG can also be used to obtain heart information by using optical technology. PPG is mainly used to measure blood oxygen saturation and can also provide information on heart function. With the help of PPG technology, heart rate monitoring can be integrated on the wearable devices for the purpose of continuous detection.

The wearable sensing device 101 is equipped with detection light, and blood pressure detection is performed by optical sensing of subcutaneous blood flow, and followed by obtaining blood pressure data through algorithm. The blood oxygen detection involves the blood volume change. Red light and infrared light sources are used to illuminate, and the light is received by the sensor through the tissue. Under the influence of the volume change, the difference on the light intensity is converted into a signal and the blood oxygen concentration is calculated. If the wearable is worn on the wrist, the light is not easy to penetrate, the reflective method is used to calculate the volume change of blood. Wearable blood glucose level sensing, for example, by analyzing sweat composition to infer blood sugar concentration, the user can take a measurement after walking 30 steps. The blood glucose sensor uses the analysis of the components in sweat to detect lactic acid or glucose to infer the blood glucose concentration. Compared with the traditional method of detecting blood glucose through blood, it has the advantages of non-invasive and real-time analysis.

The wearable sensing device 101 includes inertial sensors such as accelerometers, gyroscopes (G-sensors), etc., the inertial sensors can detect various types of motion according to the configuration. The wearable sensing device 101 may be attached on any suitable body position, such as arm, wrist, thigh, calf, ankle and so on.

A wearable sensing device 101 monitors heart sounds, electrocardiograms, lung sounds, blood pressure, blood glucose level and blood oxygen saturation level of the user 10. The sensing device 101 communicates with external mobile devices 103 (for example, smart phones, tablet computers, etc.). The heart sound data collected by the wearable heart sound sensing device 101 is uploaded from the mobile device 103 to the cloud server 107 via the cloud network 105 through wireless transmission (for example, Bluetooth and WiFi). The data in the cloud server 107 will be stored in the cloud big data database 108. The above system 10 also includes an application program installed in the mobile device, the application program includes instructions for receiving and sending data between the wearable heart sound sensing device 101, the mobile device 103 and the cloud server 107. The above application program can be operated based on the Android, Windows or iOS operating system platform, and the relevant collected data/signals, such as heart sound signals and their waveforms, are uploaded to the cloud server 107 for storage. The data is processed by algorithms analyze and feature extraction to generate evaluation reports and provide medical advice based on the evaluation reports.

In the embodiment of detecting heart and lung sounds, the wearable sensing device 101 is pasted on the chest of the user 10 in the form of a monitoring patch for the convenience of wearing. It perceives the sound signal of the human body through the built-in acoustic sensor. The acoustic sensors are mainly formed with piezoelectric sensors and microphones, in which the piezoelectric sensor is mainly composed of a piezoelectric material layer (for example, polyvinylidene fluoride (PVDF) polymer piezoelectric film, lead zirconate titanate (PZT) and other materials), the upper and lower surfaces of piezoelectric sensors are plated with conductive metals (for example, aluminum (Al), copper (Cu), etc.). Leads are pulled out from the upper and lower layers of metal to connect with the circuit board to measure the voltage signal generated by vibration. The main component of the microphone is a capacitive sensor, which uses an ultra-thin material as the diaphragm (for example, 30-micron meter thick glass), it plated with conductive material, and the diaphragm is connected to the circuit board by glue, thereby encapsulating diaphragm to form a resonant chamber. The sound from the heartbeat vibrates the diaphragm to cause a capacitance change between the diaphragm and the circuit board. The heart sound capturing device captures this change and records the heartbeat signal. The heart sound sensing device 101 may adopt a microphone, the main component of the microphone is a capacitive sensor, which uses an ultra-thin material as a diaphragm including conductive material formed thereon. A resonance chamber is subsequently formed on the diaphragm.

The sport/health cloud system of the present invention includes a pressure sensing element for detecting plantar pressure. The pressure sensing element can be mainly divided into two types: capacitive type and resistive type. Resistive pressure sensing elements are composed of conductive polymers. The resistance of the conductive polymers is changed when the pressure changes. Applying force brings the conductive particles into contact, the action increases the current through the sensing element and followed by calculating the pressure. FIG. 1 shows an insole sensing device 109 with a sandwich structure sensor according to one embodiment of the present invention. The pressure sensing layer is embedded in the insole, it includes pressure sensors with an array configuration, and individual pressure sensors are used as sensing points for sensing pressure changes and pressure distribution. The pressure sensor is electrically connected to the sensing module through wires. In one embodiment, the pressure sensor is a resistive pressure sensing element, and the resistive pressure sensing element is composed of a conductive polymer. As mentioned above, applying force may increase the current for calculating the pressure. Another embodiment of the pressure sensor is a capacitive pressure sensing element. The capacitive pressure sensing element uses a diaphragm with two separate conductive plates. When the diaphragm on the sensing element is deformed by pressure, the gap between the two conductive plates is changed and it further causes a capacitance change, the pressure is determined based on the change in capacitance. In an embodiment, the present invention may also include an acceleration sensor, a gyroscope or other sensors.

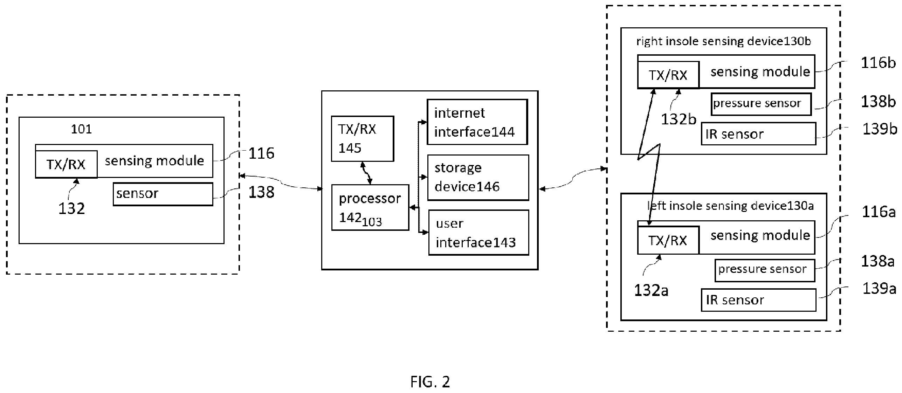

FIG. 2 shows a functional block diagram of the present invention. According to FIG. 2 and related embodiments, as shown in the figure, a wearable sensing device 101 and an insole sensing device 109 are respectively and electrically connected to a mobile device 103, such as a smart phone or a tablet computer.

Referring to FIG. 2, the wearable sensing device 101 includes a sensor 138 and a sensing module 116. The wearable sensing device 101 obtains at least one type of signal such as heart sound, electrocardiogram, lung sound, blood pressure, blood glucose level and blood oxygen saturation level from the human body through the sensor 138 and followed by transmitting the signal to the sensing module 116. The sensing module 116 receives and send data through the wireless transmission/reception module 132. The wireless transmission/reception module 132 is compatible with wireless communication standards, such as WiFi, Bluetooth, RFID, NFC, 5G or any other future wireless communication specifications, the wireless transmission module 132 is connected to the antenna to send data and receive data.

The sensing module 116 executes software applications, which includes a microprocessor and a storage unit. The microprocessor may be a microcontroller, digital signal processor (DSP), application specific integrated circuit (ASIC), programmable logic circuit or other digital data processing device that executes instructions to perform processing operations according to the present invention. The microprocessor can execute various application programs stored in the storage unit, including executing firmware algorithms. The storage unit may include read only memory (ROM), random access memory (RAM), electrically erasable programmable ROM (EEPROM), flash memory, or any memory commonly used in computers.

FIG. 2 shows left and right insole sensing devices 130a, 130b, respectively. The modules 132a, 132b perform data transmission/reception to/from the foot sensing modules 116a, 116b. In one embodiment, the wireless transmission/reception modules 132a, 132b may be Bluetooth, WiFi or similar wireless data transmission/reception devices. In other words, the aforementioned wireless transmission/reception modules 132a, 132b are compatible with wireless communication standards, such as WiFi, Bluetooth, RFID, NFC, 5G or any other future wireless communication standards. The foot sensing modules 116a, 116b are electrically connected to a plurality of pressure sensors 138a, 138b and/or infrared sensors 139a, 139b disposed on the insole via connection terminals. The foot sensing modules 116a, 116b also include processing systems (one or more microprocessors), memory, additional sensors (including accelerometers, gyroscopes (G-sensors), GPS, etc.) and a power supply device to supply power to each component. It should be understood that the foot sensing modules 116a, 116b provide computer programs/algorithms to control the collection and storage of data (for example, the pressure distribution data of the user's feet or the interaction pressure data between feet and the ground, the user's foot blood circulation status, etc.), and these programs/algorithms are executed by the processing systems.

In FIG. 2, the left and right insole sensing devices 130a, 130b communicate with the external mobile device 103. The left and right insole sensing devices 130a, 130b each include a foot sensing module 116a, 116 embedded in the arch of the insole, electrically connected to the pressure sensors 138a, 138b and the infrared sensors 139a, 139b to receive and analyze the user's foot pressure distribution and foot blood circulation data. Through the wireless transmission/reception (TX/RX) modules 132a, 132b located in the foot sensing modules 116a, 116, the data is transmitted to a remote computing device or server. The foot sensing modules 116a, 116 respectively include a processing system (one or more microprocessors), memory, additional sensors, and a power supply.

The mobile device 103 includes a processor 142, a user interface 143, an internet interface 144 and a storage device 146, respectively connected to the processor 142. The user interface 143 includes one or more input devices (eg, keyboard, touch screen, voice input device, etc.), one or more audio output devices (eg, speaker, headphone jack, etc.), and/or one or more visual output devices (such as video graphics displays, touch screens, etc.). The internet interface 144 includes one or more networked devices, such as wireless local area network (WLAN) devices, wired LAN devices, wireless wide area network (WWAN) devices, etc. The storage device 146 includes flash memory, one or more hard disk drives, one or more solid-state (SS) storage devices and/or cloud storage.

In one embodiment, the big data database 108 is connected to the cloud server 107 (FIG. 1), and the big data database 108 is electrically connected to the AI algorithm module 148. In one embodiment, AI comparison and classification are carried out through the AI algorithm module 148 installed in the cloud server 107 to analyze the information data collected by the big data database 108. The AI algorithm includes a series of steps, for example, pre-filtering and normalizing the input signal, extracting time-domain and frequency-domain features, and using a convolutional neural network (Convolutional Neural Networks, CNN) model to output classification results. Similarly, the cloud server 107 includes a user interface 143a, an internet interface 144a, a storage device 146a, and a processor 142a.

In one embodiment, no matter whether it is an uphill or downhill run or exercise, the mobile device 103 may classify the exercise intensity through the accurate sensing data of the insole, so as to facilitate appropriate exercise training and avoid sport injuries and accidents. It is suitable for all kinds of sports: walking, jogging, running, jumping. After AI analysis through the user's weight, speed, energy consumption, pressure and other data, the exercise intensity is graded by the present invention. The above benefits and functions cannot be achieved by conventional sport watches.

In another point of view, the traditional exercise management lacks visual-based learning advices. In addition to the average speed, the present invention also provides real time or specific time instant speed, so that the training management is more flexible. The present invention provides accurate data per second for competitive sports such as running, the users can clearly understand every detail of the sport state. It assists the users in training programs such as running pace and sprinting. It can also be used in the training management of other sports, such as basketball, football, badminton, long jump, etc. The present invention provides every detailed trajectory during the exercise process, and performs trajectory comparison, the user may adjust the training appropriately based on the data. The present invention also provides exercise challenge mechanisms to offer group sport for user and friends. Group sport records can be used as competitions to enhance the pleasure of sports.

The present invention integrates smart health sensing solutions, cloud big data management, and remote health management systems. Combining artificial intelligence (AI), Internet of Things (IoT) and pressure-sensing micro-microphone technology, a comfortable and extremely thin wearable sensing product is designed, which can sense and record physiological (such as heart sound) status 24 hours a day, providing users with health status trends. The present invention uses AI algorithms to analyze heart sound data to generate health management reports, grasp health status and trends for self-health management. When an abnormality or emergency occurs (such as ventricular fibrillation, etc.), an alarm will be sent immediately to the emergency medical services.

The mobile device 103 processes the data to produce various results. For example, the data from the sensors in the shoe is utilized to analyze the pressure distribution, the weight ratio of the feet, gait, stride frequency, the center of pressure (COP), and any information related to foot pressure. The feet pressure distribution plays a key role in human movement. Foot shape and walking (running) posture affect the body posture, as well as the athlete's performance. The insole with integrated interlayer sensor proposed by the present invention can obtain the parameter data of the foot pressure distribution of users with respect to time and space, and the data may be upload to the external device (for example, smartphones, personal computers, computer servers, etc.) through wireless transmission devices and the data is subsequently stored in big data databases.

In addition, the insole disclosed by the present invention also integrates an infrared detection device to simultaneously provide information on the user's blood circulation status. Break through the limitation that only medical or sports research institutions can obtain data analysis, more diverse sports and more users may obtain exclusive personal movement or exercise analysis. The collected data also shared by various professional entities, such as sport center, medical care, shoemaking industry. These entities may fetch the foot pressure data to offer further service.

In one embodiment, the aforementioned data is transmitted wirelessly, and is displayed by application program APP in real time, the data is therefore visualized. The visualized data enables more users to have individual sport analysis. Various professional entities may utilize the exercise and foot pressure data, and thereby promoting further development of various algorithms and apps, thereby unlocking the mysteries of human movement and posture.

The internet of things wearable stethoscope of the present invention is connected to the mobile device 103, and it provides 24H internet real-time monitoring. If there is no mobile device 103 connected, the heart sound information may be recorded offline. After the mobile device 103 uploads the information to the server 107, it is analyzed by the AI module 48, and the analysis results are subsequently displayed on the screen through the application program APP of the mobile device 103, the analysis results are indicated with normal, abnormal, and emergency states, respectively. For the abnormal and emergency states, a warning signal is issued through the mobile device 103. For example, the arrhythmia is an abnormal situation, the system will suggest seeking medical treatment. Examples of emergency states are ventricular fibrillation and cardiac arrest. At these situations, the APP not only sends out a warning, but also notifies emergency contacts, ambulance services, police departments, insurance companies, etc. Therefore, the user may designate medical services, emergency contacts by the aforementioned APP for emergency rescue. In one embodiment, the user may set up the emergency contacts and the ambulance services in advance.

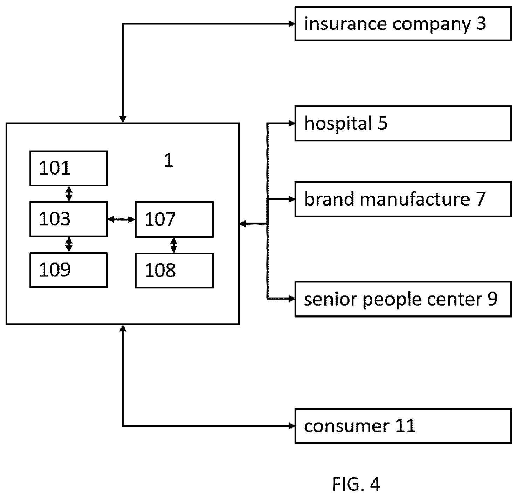

FIG. 4 shows the embodiment of the present invention. The sport health cloud system 10 of the present invention includes a cloud server 107 electrically connected to a cloud big data database 108. The wearable sensing device 101 and the sensing insole 109 are connected to the mobile device 103. The analysis data stored in the big data database 108 of the present invention provides health management, the system 10 may be cross-industry combination with various businesses, such as hospitals, insurance companies, senior centers, brand manufacturers, etc. For example, the information is uploaded to the cloud via mobile phones, and AI is used to analyze the big data for providing accurate sport and health analysis which are utilized by consumers, insurance companies and hospitals, so as to facilitate risk analysis and medical treatment evaluation. In addition, the big data database 108 uses the block chain as the communication mechanism, the data will not be altered and the transmission is encrypted. Furthermore, AI analysis of big data may provide a customized service platform to cooperate with international manufacturers/hospitals/insurance companies. The present invention establishes sport coaching models, group competitions and other gamified apps to promote sports as games and thereby increasing the network traffic.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. A sport and health cloud system (1), wherein the improvement comprising:
a cloud server (107) including an intelligent analysis module (148);
a big data database (108) electrically coupled to said cloud server (107);
a wearable sensing device (101) for collecting at least one of heart sounds, electrocardiograms,
lung sounds, blood pressure, blood glucose level and blood oxygen saturation level;
an insole-type sensing device (109) for collecting at least foot information; and
wherein said wearable sensing device (101) and said insole sensing device (109) are connected to a mobile device (103) connected to said cloud server (107) for transmitting collected data which is analyzed by said intelligent analysis module (148) to obtain health or exercise data for facilitating the assessment of health status or exercise intensity.

2. The system of claim 1, wherein said wearable sensing device (101) includes an internet of things wearable stethoscope connected to said mobile device for real-time monitoring online, or recording heart sound information offline

3. The system of claim 2, wherein mobile device (103) displays analyzed heart sound information which is indicated with a normal, an abnormal or an emergency state, and issues a warning for said abnormal and said emergency states through said mobile device.

4. The system of claim 3, wherein said mobile device (103) is set in advance with an emergency contact person, an ambulance service, a police department or an insurance company.

5. The system of claim 4, wherein said setting is used for designating a medical service, emergency contact, an emergency rescue or a rescue network.

6. The system of claim 1, wherein said foot information is displayed in real time by said mobile device (103) to analyze a personal movement or exercise, and establish a relationship between said exercise and a foot pressure.

7. The system of claim 1, wherein said insole-type sensing device (109) includes a pressure sensor (138a, 138b), an infrared sensor (139a, 139b) or combination thereof.

8. The system of claim 7, wherein said insole-type sensing device (109) includes an accelerometer, a gyroscope, a GPS or any combination thereof.

9. The system of claim 8, wherein said analyzed data includes one or any combination of following: a foot pressure distribution, a body weight ratio between right and left foot, a gait, a stride frequency and a pressure center.

10. The system of claim 1, wherein said data stored in said mobile device (103) or said big data database (108) is used for health management or used by a third party.

11. The system of claim 10, wherein said third party includes a hospital (5), an insurance companies (3), a senior people centers (9) or a brand manufacture (7).

12. The system of claim 112, wherein said big data database (108) employs a block chain technology.

13. The system of claim 1, wherein said data is analyzed by said intelligent analysis module (148) to obtain sport and health information.

14. The system of claim 13, wherein said sport and health information is used by an insurance company (3) or a hospital (5) for insurance risk analysis, or hospital treatment evaluation.

15. The system of claim 1, further including collecting exercise information, wherein said exercise includes at least one of foot, ankle, knee or hand exercise.

## Amended claims

### Amended claims in accordance with Rule 137(2) EPC.

1. A sport and health cloud system (1), wherein the improvement comprising:
a cloud server (107) including an intelligent analysis module (148);
a big data database (108) electrically coupled to said cloud server (107);
a wearable sensing device (101) for collecting at least one of heart sounds, electrocardiograms, lung sounds, blood pressure, blood glucose level and blood oxygen saturation level;
an insole-type sensing device (109) including a pressure sensor (138a, 138b), an accelerometer, a gyroscope for collecting at least foot information; and
wherein said wearable sensing device (101) and said insole sensing device (109) are connected to a mobile device (103) connected to said cloud server (107) for transmitting collected data which is analyzed by said intelligent analysis module (148) to obtain health or exercise data for facilitating the assessment of health status or exercise intensity, wherein analyzed data includes one or any combination of following: a foot pressure distribution, a body weight ratio between right and left foot, a gait, a stride frequency and a pressure center.

2. The system of claim 1, wherein said wearable sensing device (101) includes an internet of things wearable stethoscope connected to said mobile device for real-time monitoring online, or recording heart sound information offline

3. The system of claim 2, wherein mobile device (103) displays analyzed heart sound information which is indicated with a normal, an abnormal or an emergency state, and issues a warning for said abnormal and said emergency states through said mobile device.

4. The system of claim 1, wherein said foot information is displayed in real time by said mobile device (103) to analyze a personal movement or exercise, and establish a relationship between said exercise and a foot pressure.

5. The system of claim 1, wherein said insole-type sensing device (109) includesan infrared sensor (139a, 139b).

6. The system of claim 5, wherein said insole-type sensing device (109) includes a GPS.

7. The system of claim 1, wherein said data is analyzed by said intelligent analysis module (148) to obtain sport and health information.
